# EUROPÄISCHE PATENTSCHRIFT

(11) **EP 1 219 244 B1**
(45) Veröffentlichungstag und Bekanntmachung des Hinweises auf die Patenterteilung: **15.09.2004**
(21) Anmeldenummer: 02006254.3
(22) Anmeldetag: 09.02.1998
(51) Int. Cl.: A61B 6/14

(54) **Verfahren und Einrichtung zur Herstellung von Röntgenaufnahmen von Körperteilen eines Menschen**
Method and apparatus for producing X-ray exposures of human body parts
Procédé et appareil pour produire des radiographies des parties du corps humain

(30) Priorität: 17.02.1997 DE 19706102
(43) Veröffentlichungstag der Anmeldung: 03.07.2002
(62) Teilanmeldung aus: 98102209.8
(73) Patentinhaber: Sirona Dental Systems GmbH, 64625 Bensheim (DE)
(72) Erfinder: Zeller, Uwe, 61267 Neu-Anspach (DE); Günther, Werner, 64625 Bensheim (DE); Schulze-Ganzlin, Ulrich, 64653 Lorsch (DE); Döbert, Michael, 64653 Lorsch (DE)
(74) Vertreter: Sommer, Peter

(56) Entgegenhaltungen:
- EP-A- 0 229 971
- EP-A- 0 632 994
- US-A- 4 188 537
- US-A- 5 600 699

## Beschreibung

Die Erfindung bezieht sich auf ein Verfahren und eine Vorrichtung zur Erstellung von Röntgenaufnahmen von Körperteilen eines Menschen, insbesondere von Röntgen-Schichtaufnahmen vom Kiefer oder Schädel eines Patienten, bei dem ein von einer Strahlenquelle erzeugtes und durch eine Blendenöffnung einer Primärblende begrenztes Strahlenbündel nach Durchdringung des Aufnahmeobjektes auf eine Detektoranordnung trifft, die mindestens einen röntgenbilderfassenden Detektor aufweist.

In der zahnärztlichen Röntgentechnik werden Verfahren und Vorrichtungen angewendet, mit denen es möglich ist, Schichtaufnahmen von einem Menschen, insbesondere vom Bereich des Kiefers, zu erstellen. Eine besondere Anwendung liegt in der Erstellung von Schichtaufnahmen, deren Schichtverlauf senkrecht zum Kieferbogen verläuft. Solche Schichtaufnahmen werden auch Transversalschnitte genannt. Im Vergleich zu den sonst üblichen Panorama-Schichtaufnahmen weisen solche Transversalschnitte einen besonders kleinen Tiefenschärfenbereich auf. In der EP-0 229 971 wird eine Vorrichtung, mit der solche Schichtaufnahmen auf einem Röntgenfilm möglich sind, aufgezeigt.

In der EP-0 632 994 wird eine Vorrichtung zur Erstellung von Röngenaufnahmen auf digitalem Weg beschrieben. Hierzu ist eine Zeilendetektorkamera mit einem Detektor vorgesehen, der als ein- oder mehrteiliger CCD-Sensor ausgeführt ist. Die Abmessungen der Detektoranordnung betragen, unabhängig davon, ob ein ein- oder mehrteiliger Sensor verwendet wird, typischerweise 135 bis 180 mm in der Bildhöhe und ca. 6 mm in der Bildbreite. Diese Maße berücksichtigen einerseits die für eine gute Diagnose notwendige Bilderfassungsgröße, andererseits eine ausreichende Tiefenschärfe bei Betrachtung der einzelnen Schichten. In der Praxis hat sich gezeigt, daß das Blendenund Detektorsystem so abgestimmt sein muß, daß der nutzbare Strahlenfächer für transversale Schichten, also für die obengenannten Transversalschnitte, eine Breite von mindestens 20 mm in Detektorebene aufweisen muß, um einen Tiefenschärfenbereich von ca. 1 bis 3 mm erzielen zu können. Eine Detektoranordnung in der vorgenannten Größenordnung ist mit der heutigen Technologie vergleichsweise teuer.

Aus der US 4,188,537 ist eine dentale Röntgeneinrichtung bekannt, bei der ein sich in vertikaler Richtung erstreckender Zeilensensor oder ein einzelner Sensor zur Erstellung einer Röntgenaufnahme in vertikaler Richtung entlang einer Linie bewegt wird. Der Sensor kann dabei auch einer Drehbewegung folgen, so dass zusammen mit der vertikalen Bewegung der gesamte Bereich des Patienten durch Röntgenstrahlen erfaßt wird.

Ziel der vorliegenden Erfindung ist es, ein Verfahren und eine Vorrichtung zu schaffen, mit der es möglich ist, die eingangs erwähnten Schichtaufnahmen mit einer kleineren, kostengünstigeren Detektorfläche erstellen zu können.

Die Aufgabe wird mit den Mitteln des Anspruch 1 gelöst. Dadurch, daß die Aufnahmeprozedur in mehreren, zeitlich getrennten Abschnitten erfolgt, zu denen der Detektor bzw. eine aus mehreren Detektoren bestehende Detektoranordnung in verschiedenen Positionen angeordnet wird, kann ein sehr viel kleinerer und damit kostengünstigerer Detektor eingesetzt werden. Besonders wirtschaftlich ist es, wenn der verwendete Detektor die Abmessung des Detektors eines sog. Intraoral-Sensors aufweist, wie er heute für Intraoral-Aufnahmen verwendet wird, oder wenn ein kompletter solcher Intraoral-Sensor dafür eingesetzt wird. Derartige Sensoren haben typischerweise Abmessungen von etwa 30 x 20 mm. Mit der zuletzt genannten Variante läßt sich der an sich für Intraoral-Aufnahmen bereits eingesetzte Sensor auch für extraorale Aufnahmen verwenden (dual use).

Gemäß der Erfindung wird eine Detektorverschiebung erst dann vorgenommen, wenn alle Transversal-Schichtaufnahmen für eine Position, also für eine Teilaufnahmeposition der Detektoranordnung durchgeführt worden sind.

Dabei kann es vorteilhaft sein, die Teilaufnahmen nicht nur in der Vorlaufphase sondern auch in der Rücklaufphase der die Röntgenstrahlenquelle und die Detektoranordnung tragenden Dreheinheit durchzuführen. Der gesamte Aufnahmeablauf läßt sich so wesentlich beschleunigen.

Vorteilhaft kann es auch sein, mehrere Detektoren vorzusehen und diese beabstandet anzuordnen. Eine solche Anordnung mit mehreren Detektoren ist immer noch kostengünstiger als eine Detektoranordnung in der eingangs genannten Bilderfassungsgröße.

Anhand der Zeichnung werden mehrere Ausführungsbeispiele der Erfindung näher beschrieben. Es zeigen:
Figur 1 ein zahnärztliches Röntgendiagnostikgerät mit der erfindungsgemäßen Vorrichtung in einer Seitenansicht,
Figur 2 eine Prinzipskizze zur Erläuterung der mechanischen Zusammenhänge der Vorrichtung,
Figur 3 eine Ausführungsform einer Detektoranordnung,
Figuren 4 und 5 zwei Versionen einer Detektoranordnung,
Figur 6 eine Prinzipskizze zur Erläuterung von Transversalschnitten,
Figur 7 ein Blockschaltbild,
Figur 8 eine weitere Ausführung einer Detektoranordnung.

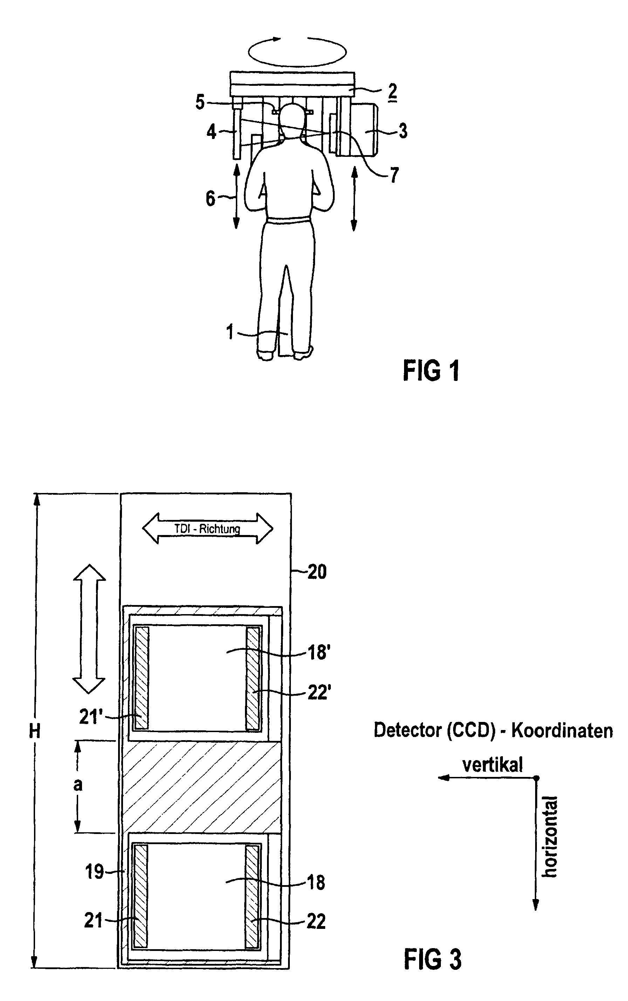

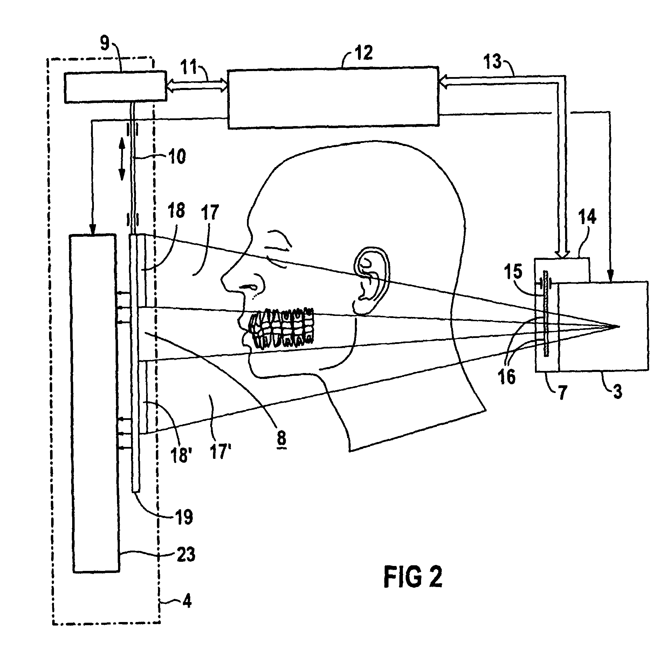

Die Figur 1 zeigt in einer Prinzipdarstellung ein zahnärztliches Röntgendiagnostikgerät zur Erstellung von Panorama-Schichtaufnahmen, welches gemäß der Erfindung auch zur Darstellung von Transversalschnitten eingesetzt werden kann. Das Gerät enthält eine in der Höhe verstellbare Tragsäule 1, an der eine Dreheinheit 2 gehaltert ist, die Träger einerseits einer Röntgenstrahlenquelle 3 und andererseits diametral dazu einer Röntgen-Detektorkamera 4 ist. Mit 5 ist eine Kopfhalteund Positioniereinrichtung bezeichnet, mit der in bekannter Weise der Patientenkopf in einer definierten Position fixiert werden kann. Aufbau sowie Verstellmöglichkeiten der Dreheinheit und der Kopfhalte- und Positioniereinrichtung sind bekannt und beispielsweise in der eingangs genannten EP-0 632 994 beschrieben. Die Zeilenkamera 4 besteht aus einem länglichen Gehäuse mit einem nicht näher bezeichneten Schlitz an der der Strahlenquelle zugewandten Seite. Hinter dem Schlitz befindet sich im Innern der Kamera eine Detektoranordnung 8 mit einem oder mehreren strahlenempfindlichen Detektoren, z.B. in Form von CCD-Sensoren. Aufbau und Anordnung werden später noch näher erläutert. Die Detektoranordnung 8 ist innerhalb der Zeilendetektorkamera in Richtung ihrer Längsachse in Richtung des Pfeiles 6 verstellbar gehaltert. Im folgenden wird von einer Detektoranordnung mit zwei aktiven Detektorelementen ausgegangen. Diese Anordnung stellt nur eine von mehreren denkbaren, im Rahmen der Erfindung liegenden Ausführungsformen dar. Synchron dazu ist auch ein mit 7 bezeichnetes Blendensystem, welches die Primärblende beinhaltet, verstellbar gehaltert. Die elektromechanische Verbindung der Zeilendetektorkamera 4 mit dem Blendensystem 7 wird anhand der Figur 2 näher erläutert.

Die im Innern angeordneten, später noch näher erläuterten Detektorelemente können mittels einer geeigneten Verstelleinrichtung, hier mittels eines Schrittmotors 9 und einer Spindel 10, entlang der Detektorhauptachse verstellt werden. Der Schrittmotor 9 kommuniziert über eine (serielle) Schnittstelle 11 mit einer Steuerelektronik 12 der Gerätesteuerung des Röntgengerätes. Die Steuerelektronik 12 gibt über eine weitere Schnittstelle 13 Steuerbefehle an einen an der Röntgenstrahlenquelle 3 angeordneten Stellantrieb 14. Mit diesem Stellantrieb erfolgt die synchrone Verstellung einer Primärblende 15 des Blendensystems 7. Die Primärblende 15 enthält zwei beabstandet angeordnete Blendenöffnungen 16. Die von der Strahlenquelle 3 erzeugten Röntgenstrahlen werden so in zwei Strahlenbündel 17, 17' unterteilt, die so fokussiert sind, daß sie exakt auf zwei im Innern der Zeilendetektorkamera 4 angeordnete Detektoren 18, 18' treffen. Die beiden Detektoren 18, 18' sind auf einem Träger 19 angeordnet, der, wie beschrieben, mittels der Verstelleinrichtung 9, 10 in der angegebenen Pfeilrichtung verstellbar ist.

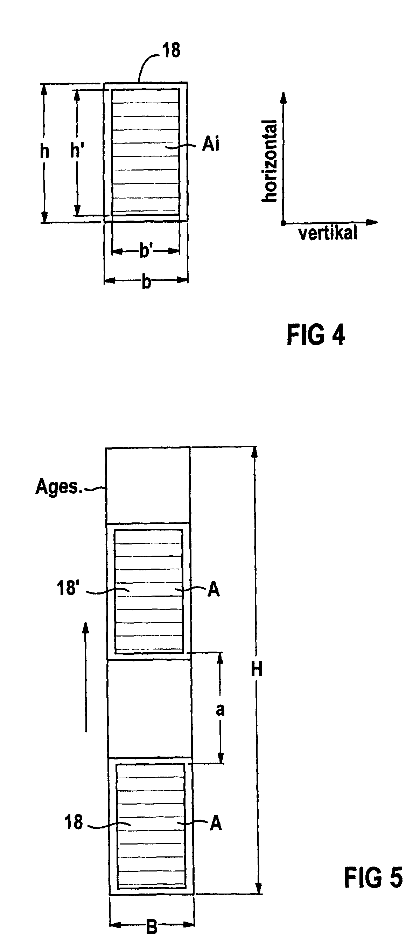

Die Figur 3 zeigt die Detektoranordnung in einer Frontansicht. Der Träger 19, auf dem die beiden Detektoren 18, 18' 9 befestigt sind, ist in einem Rahmen 20 verschiebbar gehaltert. Mit 21 bzw. 21' sind die Austaktregister der Detektoren bezeichnet, die, wie aus der Koordinatenbezeichnung rechts im Bild ersichtlich, als Horizontalregister ausgebildet sind, d.h. die TDI-Richtung (TDI steht für Time Delay and Integration und ist eine CCD-spezifische Technologie) verläuft quer zur Verschieberichtung. Mit 22, 22' sind zweite Ausleseregister bezeichnet, die gemäß einer vorteilhaften Variante dann vorgesehen werden müssen, wenn man das Austakten in der entgegengesetzten Richtung ausführen will, wenn man also Teilaufnahmen während der Rücklaufphase der Dreheinheit machen will.

Mit 23 in Figur 2 ist die Ansteuerelektronik für die beiden Detektoren 18, 18' bezeichnet.

Figur 4 zeigt einen einzelnen Detektor 18 mit den bei Intraoral-Sensoren üblichen Abmessungen. Solche Sensoren haben typischerweise eine Höhe (h) von 30 mm und eine Breite (b) von 20 mm. Die von Strahlung ausgeleuchtete Sensorfläche ist mit Ai bezeichnet und beträgt in der Höhe (h') 26 mm und in der Breite (b') 18 mm.

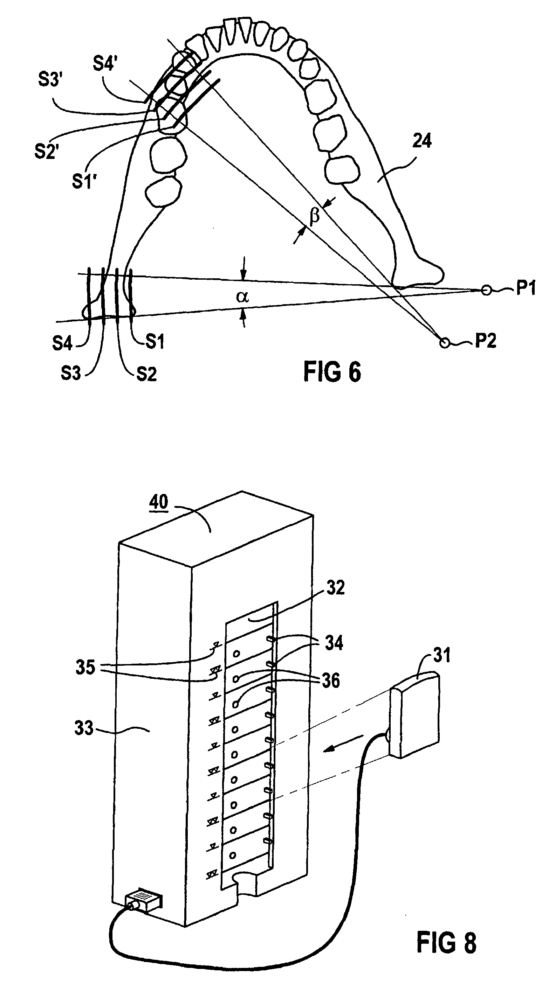

Die Figur 5 zeigt eine Detektoranordnung mit mehreren Detektoren. Die zur Objektaufnahme erforderliche Detektorfläche (Ages.)ist in n-Flächen unterteilt, wobei die Teilfläche A dem n-ten Teil der gesamten Detektorfläche (Ages.) entspricht. Jede Teilfläche setzt sich aus m-Sensorflächen (Ai) zusammen, wobei m die Anzahl der verwendeten Detektoren repräsentiert. Im Anwendungsbeispiel sind zwei Detektoren 18, 18' vorgesehen. Die aus dieser Anordnung resultierende ausgeleuchtete Teilfläche A beträgt 2 x Ai. In Relation dazu ist die gesamte, für die Aufnahme eines Objekts erforderliche Detektorfläche mit Ages. bezeichnet. Es sei hier angenommen, daß diese Gesamtfläche (Ages.) H x B = 100 mm x 20 mm betragen soll. Die beiden Detektoren 18, 18' sind im Abstand (a) voneinander am Träger 19 befestigt, wobei der Abstand a so bemessen ist, daß sich die ausgeleuchteten Teilflächen zu einem kompletten Bild ergänzen, ohne daß eine Beeinträchtigung an den Bildübergangsstellen erkennbar ist. In der beispielsweise vorgeschlagenen Anordnung werden Detektor und Strahlenfächer jeweils um ca. 25 mm in Pfeilrichtung, d.h. entlang der Längsachse der Detektoren verschoben. Um die angegebene Gesamthöhe von etwa 100 mm zu erzielen, sind demnach zwei Aufnahmephasen notwendig, eine erste in der gezeigten Grundstellung und eine zweite, bei der die beiden Detektoren um ca. 25 mm in Pfeilrichtung verschoben sind.

Alternativ kann auch eine Anordnung mit nur einem Detektor vorgesehen werden.

In diesem Falle sind vier Aufnahmephasen mit viermaliger Verschiebung des Detektors um jeweils ca. 25 mm erforderlich.

Anhand der Figur 6 wird der Ablauf zur Erzielung von Transversalschichtaufnahmen näher erläutert. Es wird dabei davon ausgegangen, daß vier Transversalschnitte in den vier angegebenen Schichtebenen 1, 2, 3 und 4 aufgenommen werden sollen.

Die Bedienperson wählt zunächst die Aufnahmeparameter (untersuchter Bereich, Anzahl, Qualität und Größe der Transversalschnitte sowie Dosis). Diese Parameter können am Röntgengerät oder an einem angeschlossenen PC durch Auswahl vordefinierter Programme oder auch durch manuelles Zusammenstellen der Parameter erfolgen. Nach Positionierung des Patienten in der Kopfhalte- und Positoniereinrichtung 5 (Fig. 1) wird der Aufnahmeablauf gestartet. Das Gerät justiert sich zunächst durch Anfahren von Referenzpunkten und positioniert sich anschließend in einer Startposition für die nachfolgende Aufnahnmeserie. Die Anzahl der Einzelaufnahmen ergibt sich durch die Anzahl der Transversal-Schichtaufnahmen und die Anzahl der Aufnahmephasen. Im Ausführungsbeispiel nach Figur 6 sei angenommen, daß von dem aufgezeichneten Kieferbogen 23 zunächst vier verschiedene Schichtaufnahmen S1 bis S4 vom linken Kiefergelenk und danach vier weitere Schichtaufnahmen S1' bis S4' von einem weiteren Kieferabschnitt erstellt werden sollen. Die Dreheinheit 2 wird zunächst in die Position P1 gefahren, von der aus von der Strahlenquelle Strahlen auf den gewünschten Abbildungsbereich abgegeben werden können, der beispielsweise den eingezeichneten Winkel α umfassen soll. Die Schichtaufnahme S1 wird erstellt, indem beginnend von einer Ausgangsposition über den Winkel α gestrahlt wird. Nachdem die Aufnahme für die Schichtlage S1 erstellt ist, wird die Strahlenquelle abgeschaltet und die Dreheinheit um den Schwenkwinkel α wieder zurück in die Ausgangsposition gefahren. Danach werden in der Folge die Schichtlagen S2, S3 und S4 erstellt. Andere Schichtlagen für weitere Objektausschnitte können analog erstellt werden, beispielsweise wie dargestellt, von der Position P2 aus. Der kinematische Bewegungsablauf der die Strahlenquelle und Zeilendetektorkamera aufnehmenden Dreheinheit ist an sich bekannt, ebenso die dazu erforderliche Ansteuerung der Detektoren nach dem TDI-Verfahren, um die gewünschte Schichtung zu erhalten.

Bei der zuletzt beschriebenen Aufnahmeprozedur wird die eigentliche Aufnahme mit Strahlung während des Vorlaufs der Dreheinheit durchgeführt. In der Rücklaufphase ist die Strahlung abgeschaltet oder sie wird durch eine Blendenverstellung unwirksam gemacht.

Um die vier Schichten im Ausführungsbeispiel erfassen zu können, gibt es mehrere Möglichkeiten. Die eine besteht darin, zunächst eine Schicht komplett zu erstellen, d.h. bei einer Detektoranordnung mit zwei Detektoren zunächst zwei Aufnahmesequenzen mit Detektorverschiebung durchzuführen und entsprechend danach die weiteren Schichten S2, S3 und S4 aufzunehmen.

Gemäß der Erfindung werden in einer Detektorposition alle vier Schichten aufgenommen, danach wird die Detektoranordnung verschoben und anschließend werden wiederum alle vier Schichten in der zweiten Detektoranordnung aufgenommen. Diese Version hat den Vorteil, daß weniger Bewegungsvorgänge notwendig sind, die unter Umständen den gesamten Ablauf beschleunigen.

Wie eingangs bereits angesprochen, kann es vorteilhaft sein, während des Rücklaufes der Dreheinheit Schichtaufnahmen durchzuführen. Dazu ist es notwendig, die Detektoren mit einem zweiten Ausleseregister (Pos. 22, 22' in Fig. 3) auszustatten, um den TDI-Prozeß auch in der entgegengesetzten Richtung ausführen zu können.

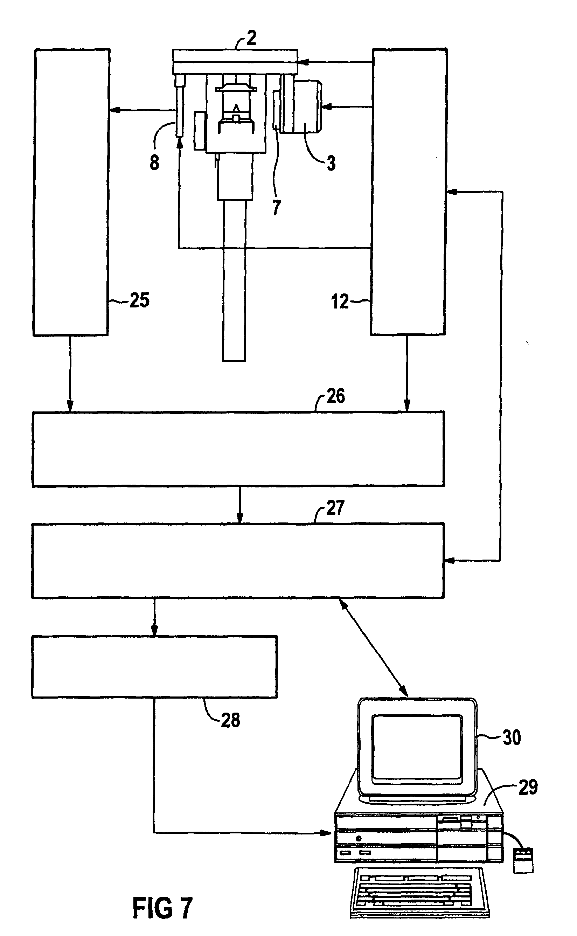

Die Figur 7 zeigt ein Blockschaltbild der erfindungsgemäßen Vorrichtung und verdeutlicht die Beziehung zwischen den einzelnen Komponenten. Die Gerätesteuerung 12 erzeugt Steuersignale für die Detektoranordnung 8, das Blendensystem 7 und Röntgenquelle 3 und koordiniert für jede Teilbildsequenz den Ablauf bzw. die Bewegung der Detektoranordnung 8 und des Strahlers 3 entsprechend der vorbestimmten Bahn bei einer Aufnahme. Die während der Sequenz erfaßten Bilddaten werden in einen Zwischenspeicher 25 abgelegt. Wie durch die Pfeilangaben erkennbar, steuert die Gerätesteuerungselektronik 12 nicht nur den Strahler 3, das Blendensystem 7 und die Detektoranordnung 8, sondern liefert gleichsam aufnahmebezogene Strahlungs-, Positions- und Bahndaten an eine Software 26. Diese verarbeitet auch die in dem Zwischenspeicher 25 abgelegten Bilddaten der Teilbilder, die mit den Detektoren gewonnen worden sind, zu einem 'Rohbild'. Dieses 'Rohbild' wird anschließend in einer weiteren Software 27 zu einem Röntgenbild verarbeitet, welches danach in einer Datenbank 28 abgelegt wird. Von dort aus kann es beispielsweise über einen PC 29 abgerufen und auf einem Monitor 30 dargestellt werden. Vom PC 29 können vorteilhafterweise durch die für eine Aufnahme notwendigen Parameter aus der Software 27 generiert werden, die von dort aus an die Gerätesteuerung weitergeleitet werden.

Die zur Erzeugung der Schichtbilder notwendige Summation bzw. Integration kann - wie aufgezeigt - mit Hilfe der CCD, die im sog. TDI-Modus beschrieben werden, erfolgen. Hierbei entsteht das Bild während der Datenerfassung durch eine 'analoge' Summation innerhalb der aktiven CCD-Fläche.

Die Bilderfassung ist alternativ auch ohne TDI-Verfahren auch möglich, indem für jedes Bewegungsinkrement entsprechend der notwendigen Sensorbewegung ein Abbild der kompletten Sensorfläche erfaßt und gespeichert wird. Für diesen Betriebsmodus wird üblicherweise der Begriff 'full frame mode' oder 'area mode' verwendet. Statt CCD-Technologie kann bei dieser Alternative auch auf eine kostengünstigere Technologie (z.B. CMOS-Technik) zurückgegriffen werden. Die zur Entstehung einer scharfen Schicht notwendige Summation von Bildpunkten erfolgt in diesem Falle nach der Datenerfassung in einer entsprechend ausgelegten Recheneinheit.

Die bei jeder Aufnahme erzeugten Signale eines Teilbildes werden nach Zwischenspeicherung von einer Signalverarbeitungssoftware zu einem Schichtbild verarbeitet. Jede Transversalaufnahme wird also aus den zugehörigen Aufnahmen von einem Rechner zusammengesetzt, bearbeitet und abgespeichert und sodann auf einem Bildschirm dargestellt. Beim Zusammensetzen sind dem Rechner die geometrischen Positionen während der Aufnahmesequenzen und auch die Abmessungen des Detektors bekannt.

Wie eingangs erwähnt, sollte die Detektorbreite mindestens 20 mm aufweisen, um Mindest-Anforderungen an die Tiefenschärfe der aufzulösenden Transversalschicht zu erfüllen. Durch Querverschieben der vorgenannten Detektorstrukturen und durch Wiederholung der beschriebenen Aufnahmeprozeduren kann die wirksame Detektorbreite vergrößert und somit die Tiefenschärfe der Transversalschicht verbessert werden.

Mit jeder Querverschiebung entsteht ein weiterer Aufnahmesatz. Diese Aufnahmesätze können, wie beschrieben, mit dem "analogen" TDI-Verfahren oder "digitale" Summation erstellt werden. Um die Querverschiebung mit zu berücksichtigen, müssen diese Aufnahmesätze ebenfalls aufsummiert werden. Zur Summation der digitalisierten Aufnahmesätze ist verständlicherweise nur das "digitale" Verfahren sinnvoll anwendbar.

Wie bereits erwähnt, erfolgt die Primärblendeneinstellung synchron zu der Verstellung der Detektoren. Dies kann durch eine motorische verschiebbare Schablone in der in Figur 2 dargestellten Weise erfolgen. Alternativ kann auch die Blende durch eine entsprechende Verstellung einer unteren und oberen Abgrenzung erfolgen; ebenso ist es denkbar, eine drehbare Blende mit an unterschiedlichen Stellen befindlichen Blendenöffnungen vorzusehen.

Die Figur 8 zeigt eine sog. 'dual-use'-Variante, bei der ein kompletter Intraoral-Sensor 31, wie er in bekannter Weise für Intraoral-Aufnahmen eingesetzt wird, auch zur Erstellung von Transversal-Schichtaufnahmen eingesetzt werden kann. Hierzu ist ein Träger 32 vorgesehen, der, wie der Träger 19 der zuvor beschriebenen Ausführung, im Gehäuse 33 der Zeilendetektorkamera 40 verstellbar angeordnet ist. Der Träger 32 enthält eine Vielzahl von Klemm- oder ähnlichen Halteelementen 34, die es ermöglichen, den Sensor 31 in verschiedenen Positionen zu haltern. Die Halteelemente 34 sind hierzu so angeordnet, daß der Sensor 31 wahlweise je nach Aufnahmeart bzw. Aufnahmebereich gehaltert werden kann. Am Gehäuse 33 angebrachte Symbol-Markierungen 35 erleichtern die Wahl des Aufnahmebereichs bzw. der Aufnahmeart. Anstelle von Symbolen kann als Markierung auch eine z.B. metrische Skalierung vorgesehen sein. Die Position des eingesetzten Sensors 31 wird durch Positionsmelder 36 erfaßt. Diese können elektromechanische Art (Mikroschalter od.dgl.) oder auch optoelektrische Art (Lichtschranken od.dgl.) sein. Die elektrische Verknüpfung und Steuerung erfolgt ansonsten wie beschrieben.

## Patentansprüche

1. Verfahren zur Erstellung von Röntgenaufnahmen in Form mehrerer Transversalschichtaufnahmen von Körperteilen eines Menschen, insbesondere von Röntgen-Schichtaufnahmen vom Kiefer oder Schädel eines Patienten, wobei ein von einer Strahlenquelle (3) erzeugtes und durch eine Blendenöffnung (16, 16') einer Primärblende (15) begrenztes Strahlenbündel nach Durchdringung des Aufnahmeobjekts auf eine Detektoranordnung (8) mit mindestens einem Detektor (18, 18') trifft, wobei die strahlungsempfindliche Fläche (Ai) des Detektors (18, 18') eine Teilfläche der zur Objektaufnahme erforderlichen Detektorfläche (Ages.) beträgt und wobei die Bildaufnahme in mehreren, zeitlich getrennten Abschnitten erfolgt, indem nach einer ersten Teilaufnahme die Detektoranordnung (8) in der Folge entlang der Längsachse und/oder Querachse der Detektorfläche verschoben wird und dazu die Blendenöffnung (16, 16') der Primärblende (15) entsprechend angepaßt wird, **dadurch gekennzeichnet, dass** eine Verschiebung der Detektoranordnung (8) erst dann erfolgt, wenn die Teilaufnahmen einer Detektorposition für alle gewünschten Schichtaufnahmen durchgeführt worden sind.

2. Verfahren nach Anspruch 1, bei dem wobei zur Erzielung mehrerer Transversalschichtaufnahmen die Teilaufnahmen in einer Vorlaufphase und/oder in einer Rücklaufphase einer die Röntgenstrahlenquelle (3) und die Detektoranordnung (8) tragenden Dreheinheit (2) durchgeführt werden.

3. Verfahren nach Anspruch 1 oder 2, bei dem als Detektor (18, 18') ein nach dem TDI-Verfahren betriebener CCD-Sensor verwendet wird.

4. Verfahren nach Anspruch 1, bei dem als Detektor (18, 18') ein Bildaufnehmer verwendet wird, der eine Sequenz von rasch aufeinanderfolgenden Flächenaufnahmen erfaßt und bei dem die zur Bildentstehung notwendige Integration bzw. Summation in einer dem Bildaufnehmer nachgeschalteten Recheneinheit durchgeführt wird.

5. Verfahren nach Anspruch 4, bei dem als Bildaufnehmer ein CCD-Sensor verwendet wird, der in der Betriebsart "full frame-mode" betrieben wird.

6. Verfahren nach Anspruch 2 und 3, bei dem als Bildaufnehmer ein CCD-Sensor verwendet wird, der in der Betriebsart der Time Delay and Integration (TDI) betrieben wird und ein als Horizontalregister ausgebildetes Austaktregister (21) aufweist, wobei die TDI-Richtung quer zur Verschieberichtung des Detektors verläuft, und wobei der verwendete Detektor (18; 31) in seiner Integrationsrichtung umschaltbar ausgebildet ist und ein zweites Ausleseregister (22) aufweist.

7. Verfahren nach einem der Ansprüche 1 bis 6, **dadurch gekennzeichnet, dass** ein einziger Detektor (31) in den Abmessungen eines Intraoralsensors verwendet ist und dass ein Träger (32) mit mehreren Halteelementen (34) zur Halterung des Sensors (31) in verschiedenen Positionen vorhanden ist, und wobei jeder Position Positionsmelder (36) zugeordnet sind, die der Position des eingesetzten Sensors (31) entsprechende Signale an eine Steuereinrichtung (12) geben.

8. Verfahren nach einem der Ansprüche 1 bis 7, **dadurch gekennzeichnet, dass** die Summation bzw. Integration der Teilaufnahmen entsprechend der Detektorebene und der gewünschten Schichtauflösung erfolgt.

## Claims

1. Method for taking x-ray images in the form of a number of transverse tomograms of parts of the human body, in particular of x-ray tomograms of the jaw or skull of a patient, a beam generated by a radiation source (3) and limited by a diaphragm aperture (16, 16') of a primary diaphragm (15) striking a detector arrangement (8) with at least one detector (18, 18') after penetrating the object to be taken, the radiation-sensitive surface (Ai) of the detector (18, 18') being a partial surface of the detector surface (Ages.) required for taking the object, and the image being taken in a number of temporally separate portions by subsequently displacing the detector arrangement (8) after taking a first partial image along the longitudinal axis and/or transverse axis of the detector surface, and for this purpose correspondingly matching the diaphragm aperture (16, 16') of the primary diaphragm (15), **characterized in that** a displacement of the detector arrangement (8) is not performed until the partial images of a detector position have been taken for all the desired tomograms.

2. Method according to Claim 1, in which in order to achieve a number of transverse tomograms the partial images are taken in a forward phase and/or in a return phase of a rotary unit (2) carrying the x-ray source (3) and the detector arrangement (8).

3. Method according to Claim 1 or 2, in which a CCD sensor operated using the TDI method is used as detector (18, 18').

4. Method according to Claim 1, in which use is made as detector (18, 18') of an image recorder which records a sequence of rapidly succeeding areal images and in the case of which the integration or summation necessary for producing an image is carried out in a computing unit downstream of the image recorder.

5. Method according to Claim 4, in which use is made as image recorder of a CCD sensor which is operated in the full frame mode.

6. Method according to Claims 2 and 3, in which use is made as image recorder of a CCD sensor which is operated in the operating mode of time delay and integration (TDI) and has a clock-out register (21) designed as a horizontal register, the TDI direction running transverse to the displacement direction of the detector, and the detector (18; 31) used being designed such that its integration direction can be switched over, and having a second read-out register (22).

7. Method according to one of Claims 1 to 6, **characterized in that** a single detector (31) is used within the dimensions of an intraoral sensor, and **in that** a carrier (32) with a number of holding elements (34) for holding the sensor (31) in various positions is present, and each position being assigned position detectors (36) which send signals corresponding to the position of the sensor (31) used to a control device (12).

8. Method according to one of Claims 1 to 7, **characterized in that** the summation or integration of the partial images is performed in accordance with the detector plane and the desired layer resolution.

## Revendications

1. Procédé pour produire des radiographies sous la forme de plusieurs radiographies de couches transversales de parties du corps d'un être humain, notamment des tomographies de la mâchoire ou du crâne d'un patient, dans lequel un faisceau de rayons produit par une source (3) de rayonnement et délimité par une ouverture (16, 16') d'un diaphragme (15) primaire rencontre un dispositif (8) de détection ayant au moins un détecteur (18, 18'), après traversée de l'objet à radiographier, dans lequel la surface (Ai) sensible au rayonnement du détecteur (18, 18') représente une surface partielle de la surface du détecteur (Ages) nécessaire à la radiographie de l'objet, et dans lequel la radiographie s'effectue en plusieurs étapes séparées dans le temps, le dispositif (8) de détection étant décalé après une première radiographie partielle dans la suite le long de l'axe longitudinal et/ou de l'axe transversal de la surface de détecteur et l'ouverture (16, 16') de diaphragme étant à cet effet adaptée de manière correspondante, **caractérisé en ce qu'**un déplacement du dispositif (8) de détection ne s'effectue que lorsque les radiographies partielles d'une position de détection ont été effectuées pour toutes les tomographies souhaitées.

2. Procédé suivant la revendication 1, dans lequel, pour l'obtention de plusieurs tomographies transversales, les radiographies partielles sont réalisées dans une phase d'avancée et/ou dans une phase de retour d'une unité de rotation (2) qui supporte la source (3) de rayonnement radiographique et le dispositif (8) de détection.

3. Procédé suivant la revendication 1 ou 2, dans lequel il est utilisé comme détecteur (18, 18') un capteur CCD entraîné par le procédé TDI.

4. Procédé suivant la revendication 1, dans lequel, il est utilisé comme détecteur (18, 18'), un moyen de saisie d'images qui détecte une séquence de radiographies de surface se succédant rapidement et dans lequel l'intégration ou la sommation nécessaire à la formation d'image s'effectue dans une unité de calcul en aval.

5. Procédé suivant la revendication 4, dans lequel il est utilisé, comme moyen de saisie d'images, un capteur CCD, qui fonctionne dans le type de fonctionnement "full frame-mode".

6. Procédé suivant la revendication 2 et 3, dans lequel il est utilisé, comme moyen de saisie d'images, un capteur CCD, qui fonctionne dans le mode de fonctionnement du TDI (Time Delay and Integration) et comporte un registre de synchronisation (21) formé en tant que registre horizontal, la direction TDI s'étendant transversalement à la direction de déplacement du détecteur, et dans lequel la direction d'intégration du détecteur (18; 31) utilisé peut être commutée et le détecteur comporte un deuxième registre (22) de lecture.

7. Procédé suivant l'une des revendications 1 à 6, **caractérisé en ce que** un détecteur (31) unique est utilisé dans les mesures d'un capteur intraoral et **en ce qu'**il y a un support (32) ayant plusieurs éléments (34) de retenue, pour la retenue du capteur (31) dans différentes positions, et dans lequel à chaque position est associé un capteur (36) de position, qui fournit des signaux correspondant à la position du capteur (31) utilisé à un dispositif (12) de commande.

8. Procédé suivant l'une des revendications 1 à 7, **caractérisé en ce que** la sommation ou l'intégration des tomographies partielles s'effectue en fonction du plan de détection et de la résolution de couche souhaitée.
